# EUROPEAN PATENT SPECIFICATION

(11) **EP 0 979 645 B1**
(45) Date of publication and mention of the grant of the patent: **12.04.2006**
(21) Application number: 99113225.9
(22) Date of filing: 08.07.1999
(51) Int. Cl.: A61K 8/49, A61K 8/896, A61Q 17/04

(54) **Light screening composition containing a polysiloxane type UV-B screening agent and a benzimidazol type UV-B screening agent**
Lichtschutzmittelzusammensetzung enthaltend ein UV-B-Lichtschutzmittel vom Polysiloxan-Typ und ein UV-B-Lichtschutzmittel vom Benzimidazol-Typ
Composition de protection solaire contenant un agent anti UV-B de type polysiloxane et un agent anti UV-B de type benzimidazole

(30) Priority: 16.07.1998 EP 98113311
(43) Date of publication of application: 16.02.2000
(73) Proprietor: DSM IP Assets B.V., 6411 TE Heerlen (NL)
(72) Inventor: Gonzenbach, Hans Ulrich, 1202 Geneva (CH); Huber, Ulrich, 8703 Erlenbach (CH); Schwarzenbach, Rolf, 8405 Winterthur (CH)
(74) Representative: Schwander, Kuno

(56) References cited:
- EP-A- 0 848 945
- EP-A- 0 860 165
- EP-A- 0 897 716
- DE-A- 19 704 990

## Description

The invention relates to photostable cosmetic light screening compositions for the protection of the human epidermis against ultraviolet rays of wavelengths between 280 and 320 nm (UV-B).

In particular the invention relates to cosmetic light screening compositions containing a lipophilic polysiloxane type UV-B screening agent and a hydrophilic benzimidazole type UV-B screening agent.

In the European patent publication EP-0813857 a sun protection composition having a synergistic UV-protection activity is described comprising a water phase; a first oil phase; a second oil phase; an emollient for the emulsion type oil in water and another emollient for the emulsion type water in oil; a hydrophilic UV-A filter like e.g. benzophenone derivatives or a hydrophilic UV-B filter like e.g. benzimidazole derivatives and a lipophilic UV-A filter like e.g. 4-tert. butyl-4'-methoxydibenzoylmethane (PARSOL 1789®) or a lipophilic UV-B filter like e.g. octocrylene (UVINUL N-539®) or an organosiloxane filter as described in WO 93/04665 or an organosiloxane filter of the benzotriazole type as described in WO94/06404, said sun protection composition is characterized in that the first and second oil phases are incompatible.

The above mentioned European publication only mentions in general that the combined filter substances have a synergistic ultraviolet protection activity without giving any guidance to select a specific filter combination to reach a specific synergistic effect. Furthermore, the organosiloxanes as described in WO 93/04665 or WO94/06404 do not specifically refer to organosiloxanes containing a chromophore residue of the benzmalonate type.

It has now been found that a sunscreen composition containing a polysiloxane having a chromophore residue of the benzmalonate type as lipophilic UV-B screening agent and a hydrophilic benzimidazol type UV-B screening agent provides synergistically enhanced protection indices.

More particularly the present invention is concerned with a cosmetic light screening composition containing in an aqueous phase about 0.5 to 10 wt% of 2-phenylbenzimidazol-sulphonic acid or a salt thereof, and at least one fatty phase, comprising about 1 to 30 wt% of a linear or cyclic polysiloxane compound of the general formula Ia or Ib, 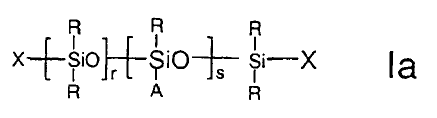 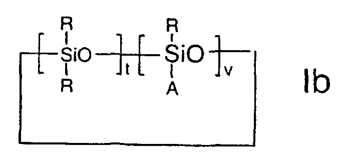 wherein
- X: signifies R or A;
- A: signifies a group of the formula IIa, IIb or IIc; 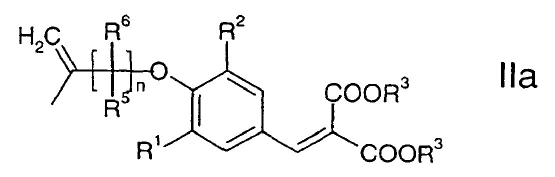 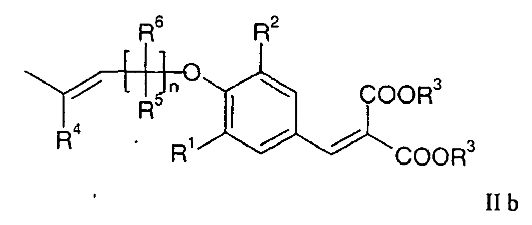 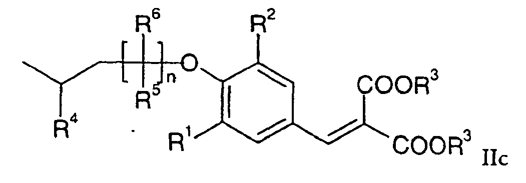
- R: signifies hydrogen, C₁₋₆ alkyl or phenyl;
- R¹ and R²: each independently signify hydrogen, hydroxy, C₁₋₆-alkyl or C₁₋₆-alkoxy;
- R³: signifies C₁₋₆-alkyl;
- R⁴: signifies hydrogen or C₁₋₆-alkyl;
- R⁵ and R⁶: each independently signify hydrogen or C₁₋₆-alkyl;
- r: has a value of from 0 to 250;
- s: has a value of from 0 to 20;
- r +s: has a value of at least 3;
- t: has a value of from 0 to10;
- v: has a value of from 0 to10; and
- v+t: has a value of at least 3;
- n: has a value from 1 to 6;
with the proviso that in the case that s is 0 at least one X is A.

The term "C₁₋₆-alkyl" refers to groups such as methyl, ethyl, propyl, isopropyl, butyl, sec. butyl, isobutyl, pentyl and neopentyl. The term "C₁₋₆-alkoxy" refers to the corresponding alkoxy groups.

The residues R are preferably methyl.

The residues R¹ and R² are preferably hydrogen, methoxy or ethoxy, more preferably hydrogen, or one of R¹ and R² is hydrogen and the other is methyl, methoxy or ethoxy.

The residues R³ are preferably methyl or ethyl, more preferably ethyl.

Preferably, R⁴ is hydrogen or methyl, R⁵ and R⁶ are hydrogen and n is 1.

Suitable salts of 2-phenylbenzimidazol-sulphonic acid are alkali salts, such as sodium- or potassium salts, ammonium salts, morpholine salts, salts of primary, sec. and tert. amines like monoethanolamine salts and diethanolamine salts.

The polysiloxane compounds having a group A of the general formula IIa and IIb and their preparation are described in the European Patent EP 0538431 B1. These polysiloxane compounds are preferred.

The polysiloxane compounds having a group A of the general formula IIc and their preparation are described in the European Patent EP 0358584 B1.

In the linear polysiloxane compounds according to formula Ia the chromophore carrying residue A may be connected to the end groups of the polysiloxane (X=A) or may be statistically distributed.

Linear polysiloxane compounds wherein the chromophore carrying residue A is statistically distributed are preferred. Said preferred polysiloxane compounds have at least one unit carrying the chromophore residue (s=1), preferably s has a value of from about 2 to about 10, more preferably a statistical mean of about 4. The number of r of the other silicone units present in the polysiloxane compounds is preferably about 5 to about 150, more preferably a statistical mean of about 60.

Polysiloxane compounds wherein 20% or less, preferably less than 10%, of the total siloxane units are units carrying a chromophore residue are preferred with respect to cosmetic properties.

The ratio of polysiloxane units having a chromophore residue A of the formula IIa to those having a chromophore residue A of the formula IIb is not critical. Said ratio may be about 1:1 to about 19:1, preferably about 2:1 to about 9:1, more preferably about 4:1.

The concentration of the polysiloxane compound in the cosmetic light screening composition is preferably about 2 to 20 wt%, more preferably about 5 wt%.

The concentration of 2-phenylbenzimidazol sulphonic acid or a salt thereof is preferably about 0.5 to 5 wt%, more preferably about 1 to 2 wt%.

The ratio of 2-phenylbenzimidazol sulphonic acid or a salt thereof to the polysiloxane compound as defined above is not critical. For example the ratio is about 1:1 to about 1:20, preferably about 1:5.

Thus, a preferred cosmetic light screening composition contains in an aqueous phase about 0.5 to 5 wt% of 2-phenylbenzimidazol-sulphonic acid or a salt thereof, and at least one fatty phase, comprising about 2 to 20 wt% of a linear polysiloxane compound of the general formula Ia, wherein
- X: signifies methyl;
- A: signifies a group of the formula IIa or IIb;
- R: signifies methyl;
- R¹ and R²: signify hydrogen, methoxy or ethoxy, or one of R¹ and R² is hydrogen and the other is methyl, methoxy or ethoxy;
- R³: signifies methyl or ethyl;
- R⁴: signifies hydrogen or methyl;
- R⁵ and R⁶: signify hydrogen;
- r: is about 5 to about 150;
- s: is about 2 to about 10;
- n: has a value of 1.

A more preferred cosmetic light screening composition contains and in an aqueous phase about 1 to 2 wt% of 2-phenylbenzimidazol-sulphonic acid or a salt thereof and at least one fatty phase, comprising about 5 wt% of a linear polysiloxane compound of the general formula Ia, wherein
- X: signifies methyl;
- A: signifies a group of the formula IIa or IIb;
- R: signifies methyl;
- R¹ and R²: signify hydrogen;
- R³: signifies ethyl;
- R⁴: signifies hydrogen;
- R⁵ and R⁶: signify hydrogen;
- r: is a statistical mean of about 4;
- s: is a statistical mean of about 60;
- n: has a value of 1.

The polysiloxane compounds Ia or Ib wherein A is a residue of the formula IIa or IIb can be prepared as described in EP 0538431 B1 by silylation of the corresponding benzalmalonates according to the following reaction scheme: 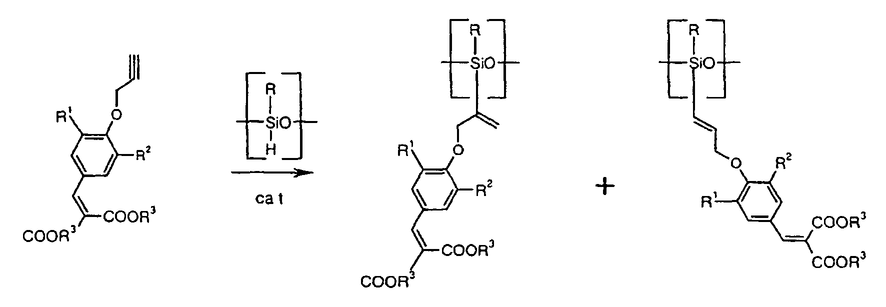 wherein R¹, R², and R³ are as defined above.

The silylation of the 4-(2-propynylox)phenyl methylene diethylester may be carried out employing known procedures for the addition of silicon bonded hydrogen atoms to groups containing aliphatic unsaturation. Such reactions are generally catalyzed by a platinum group metal or a complex of such a metal. Examples of catalysts which may be employed are platinum on carbon, chloroplatinic acid, platinum acetyl acetonate, complexes of platinum compounds with unsaturated compounds e.g. olefins and divinyl disiloxanes, complexes of rhodium and palladium compounds and complexes of platinum compounds supported on inorganic substrates. The addition reaction may be performed at reduced, atmospheric or increased pressure. A solvent can be used, e.g. toluene or xylene, in the reaction mixture although the presence of the solvent is not essential. It is also preferred to carry out the reaction at elevated reaction temperatures e.g. from about 50°C up to 150°C.

The production of the novel light screening compositions comprises incorporating a polysiloxane compound as defined above and 2-phenyl-benzimidazol-sulphonic acid or a salt thereof optionally in combination with other known UV-A and/or UV-B filters, in a cosmetic base which is usual for light screening agents.
Suitable UV B filters, i.e. substances having absorption maxima between about 290 and 320 nm, are for example the following organic compounds which belong to the widest classes of substance:
--- p-Aminobenzoic acid derivatives such as ethyl-, propyl-, butyl-, and isobutyl p-aminobenzoate;
--- Acrylates such as 2-ethylhexyl 2-cyano-3,3-diphenylacrylate (octocrylene), ethyl 2-cyano-3,3-diphenylacrylate;
--- Aniline derivatives such as methyl anilinum methosulfate;
--- Anthranilic acid derivatives such as menthyl anthranilate;
--- Benzophenone derivatives such as benzophenone-3, benzophenone-4.
--- Camphor derivatives such as methyl benzylidene camphor (PARSOL 5000), 3-benzylidene camphor, camphor benzalkonium methosulfate, polyacrylamidomethyl benzylidene camphor, sulfa benzylidene camphor, sulphomethyl benzylidene camphor and therephthalidene dicamphor sulfonic acid;
--- Cinnamate derivatives such as octyl methoxycinnamate (PARSOL MCX) or ethoxyethyl methoxycinnamate as well as cinnamic acid derivatives bond to siloxanes;
--- Gallic acid such as digalloyl trioleate;
--- Salicylate derivatives such as isopropylbenzyl salicylate, benzyl salicylate, butyl salicylate, octyl salicylate (Neo Heliopan OS), isooctyl salicylate or homomenthyl salicylate (homosalate, Heliopan);
--- Triazole derivatives such as hydroxyphenylbenztriazole, 2-2'methylene-bis-(6-(2H-benzotriazole-2-yl)-4-(1,1,3,3,-tetramethylbutyl)-phenol (TINOSORB M);
--- Triazone derivatives such as octyl triazone (Uvinul T-150), dioctyl butamido triazone (Uvasorb HEB).
--- Pigments such as microparticulated TiO₂, ZnO.

The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.

The formulation may further contain UV-A filters such as
--- Dibenzoylmethane derivatives such as 4-tert. butyl-4'-methoxydibenzoyl-methane;
--- Triazine compounds as described in the European Patent Publications EP 0693483 A1, EP 0704437 A2, EP 0704444 A1 and EP 0780382 A1, e.g. 2,2'-[6-(4-methoxyphenyl)-1,3,5-triazine-2,4-diyl]bis[5-[2-ethylhexyl) oxy]-; available under the tradename TINOSORB S from Ciba Speciality Chemicals Holding Switzerland.

As cosmetic bases usual for light screening compositions in the scope of the present invention there can be used any conventional preparation which corresponds to the cosmetic requirements, e.g. creams, lotions, emulsions, salves, gels, solutions, sprays, sticks and milks; see also, Sunscreens, Development, Evaluation and Regulatory Aspects, ed. N.Y. Lowe, N.A. Shaath, Marcel Dekker, Inc. New York and Basel, 1990.

The following examples explain the invention in more detail.

### Example 1

### Preparation of propanedioic {(4-[2-propynyloxy)phenyl]methylene} diethylester as described in EP 0538431 B1.

To a stirred suspension of 4-hydroxybenzaldehyd (425.8 g) and K₂CO₃ (807.6g) in acetone (2.96 ml) at reflux temperature of about 60°C under a nitrogen atmosphere, was added dropwise 3-bromo-propyne (502.4g) over a period of two hours. The reaction was heated at reflux for more than 3 hours. After cooling to room temperature the reaction mixture was filtered, the excess of K₂CO₃ was removed and washed several times with acetone. The filtrate was washed with saturated aqueous solution of NaHCO₃ and NaCl. The aqeous phase was extracted with diethylether. The combined organic extracts were dried over Na₂SO₄, filtered and concentrated to a volume of 1 l. The solution was kept in the refrigerator overnight. The crystals were filtered out and washed with cold diethyl ether. The filtrate was kept in the refrigerator and some more crystals were performed and removed. This procedure was repeated 3 times resulting in 1,385 g of 4-(2-propynyloxy) benzaldehyde in 83% yield. The material was analyzed by gaschromatographie, and shown to be 99,9% pure. The resulting compound (449.2 g) was added in small amounts to a stirred solution of diethylmalonate (448.5 g), piperidine (23.84 g), toluene (1,400 ml) and acetic acid (59g) at about 50°C. The acetic acid had been added in three equal portions after 1, 1.5, and 2 hours respectively. The reaction mixture was heated to reflux. After four hours the mixture was allowed to cool to room temperature and washed with saturated aqueous solution of NaHCO₃ and NaCl, dried with Na₂SO₄, filtered and concentrated, giving 853.4 g of a dark brown oily product. Diethylether (458 ml) and n-hexane (358 ml) were added and the solution was kept in the refrigerator overnight. The solution was filtered, giving 564.8 g of light brown crystals (67%yield) having a melting point of 45.5 to 48°C. Recrystallisation in ethanol and n-hexane yielded 543 g of the title compound as light brown crystals. The material was analysed by gaschromatographie, and shown to be 99,9% pure.

### Example 2

Preparation of an organosiloxane compound of the general formula Ia wherein R signifies methyl, s is 0, r is 20, X is A and A signifies a benzalmalonate residue of the formula IIa and IIb wherein R¹ and R² are hydrogen, R³ is ethyl and R⁴, R⁵ and R⁶ are hydrogen, n is 1 as described in EP 053 431 B1.

5g of {(4-(2-propynyloxy)phenyl} methylene}-diethyl ester were dissolved in 20 g of toluene and heated under nitrogen to about 80°C. 13.2 g of a hydrosiloxane having a degree of polymerization of 20 and 10 mpc SiH groups (3.62.% SiH) were then added dropwise after a platinum-divinyl-tetramethyl-disiloxane complex was also added, giving 10⁻⁴ mole of Pt per mole of SiH of the hydrosiloxane. The mixture was heated to reflux and maintained until all SiH had disappeared of the infrared spectroscopic analysis. It was then allowed to cool to room temperature. The toluene was then evaporated to leave after washing 16.5 g of a slightly brown polymer having the average structure A-[(CH₃)₂SiO]₂₀-A, wherein A is a residue of the formula IIa₁ and IIb₁,

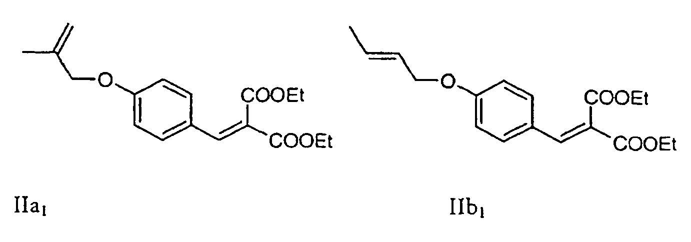

### Example 3

Preparation of an organosiloxane compound having the general formula Ia wherein R signifies methyl, r is 59, s is 4, X is methyl and A signifies a benzalmalonate residue of the formula IIa and IIb wherein R¹ and R² are hydrogen, R³ is ethyl, R⁴, R⁵ and R⁶ are hydrogen as described in the European publication EP 0709080 A1.

13.28 g of {[4-(2-propynyloxy)phenyl]methylene}-diethyl ester were dissolved in 75 g of toluene and heated under nitrogen to about 70°C. 44 g of a hydrosiloxane having a degree of polymerization of 65 and 6 mpc SiH groups (2.36% SiH) were then added dropwise after a platinum-divinyl-tetramethyl-disiloxane complex was also added, giving 10⁻⁴ mole of Pt per mole of SiH of the hydrosiloxane. The mixture was heated to reflux and maintained until all SiH had disappeared of the infrared spectroscopic analysis. It was then allowed to cool to room temperature. The toluene was then evaporated to leave after washing 52 g of a brown, viscous polymer having the average structure (CH₃)₃SiO-[(CH₃)₂SiO]₅₉-[(CH₃)ASiO]₄-Si(CH₃)₃, wherein A has the formula IIa₁ and IIb₁ 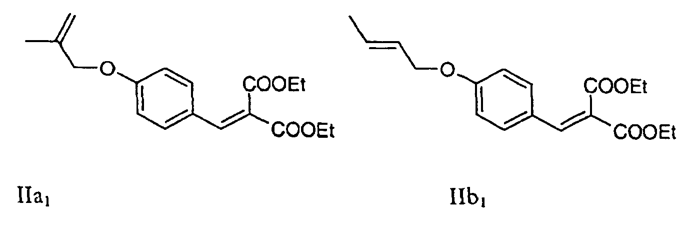 The ratio of compounds having a residue IIa₁ to compounds having a residue IIb₁ is about 4:1.

### Example 4

### Suitable cosmetic light screening compositions

A sunscreen O/W lotion comprising 5wt% of a polysiloxane of formula Ia according to Example 3 and 1wt% of 2-phenylbenzimidazol-sulphonic acid was prepared with the following ingredients:

| Part | %w/w | ingredient | CTFA Name |
|---|---|---|---|
| A | 2.5 | Arlacel 60 sold by ICI | Sorbitan Stearate |
| | 17.5 | Witconol APM sold by Witco | PPG-3 Myristylether |
| | 1.0 | Stearyl alcohol | Stearyl alcohol |
| | 5.0 | | Polysiloxane |
| | 5.0 | Silicon oil | Dimethicone 200/100 |
| B | 1.0 | PBSA | Phenylbenzimidazolsulfonicacid |
| | 1.8 | NaOH 10% | Sodium Hydroxide |
| | 2.5 | Tween 60 | Polysorbate 60 |
| | 0.3 | Keltrol sold by Kelco UK | Xanthan Gum |
| | 61.4 | Water | |
| C | 2.0 | Sepigel 305 sold by Seppic | Polyacrylamid & C13-14 isoparaffin+laureth-7 |

| | | | |
|---|---|---|---|
| CTFA: Cosmetic, Toiletry and Fragrance Association. | | | |
| Part A: Added to and melted in the reactor. | | | |
| Part B: mixed, neutralized and heated on hotplate to 85°C. | | | |
| Part C: added at 50°C. | | | |

### Example 5

### Determination of the Sun Protection Factor

The determination of the Sun Protection Factor was performed according to the COLIPA protocol (the European Cosmetic, Toiletry and Perfumery Association, Sun Protection Factor Test method, October 1994).

A solar simulator SU 2000 having a lamp intensity in the range of 5.0 to 5.5 mW/cm² was used. Irradiation area 6x1 cm²; dose progression 25%.

The following filter combinations have been formulated using the same cosmetic base as described in Example 4 to yield a stable sunscreen lotion with identical distribution characteristics.

An application dose of 2 mg/cm² was used according to the COLIPA protocol for in vivo SPF measurements in humans, on an application area of 50 cm².

As comparison the following sunscreen compositions have been tested:

A sunscreen composition containing a polysiloxane compound according to Example 3 and a second known lipophilic UV-B filter, namely 2-ethylhexyl-p-methoxycinnamate (OMC) (available under the trade name PARSOL MCX)

A sunscreen composition containing a polysiloxane compound according to Example 3 and a second known hydrophilic UV-B filter, namely the diethanolamine salt of p-methoxycinnamatic acid (MC-DEA) (available under the tradename PARSOL Hydro).

The results are given in the following Table.

| UV-Filter | in vivo SPF | expected SPF | synergy |
|---|---|---|---|
| 10wt% P3 | 4.7 | | |
| 2wt% PBSA | 6.8 | | |
| 5wt% P3 + 1wt% PBSA | 8.1 | 5.8 | 40% |
| comparative | | | |
| 10wt % P3 | 4.7 | | |
| 2wt % OMC | 5.3 | | |
| 5wt %P3 + 1wt %OMC | 5.8 | 5.0 | 16% |
| 10wt % P3 | 4.7 | | |
| 2wt % MC-DEA | 4.2 | | |
| 5wt %P3 + 1wt %MC-DEA | 4.8 | 4.5 | 7% |

| | | | |
|---|---|---|---|
| P3: Polysiloxane according to Example 3. | | | |
| PBSA: 2-phenylbenzimidazol-sulphonic acid | | | |
| OMC: 2-ethylhexyl-p-methoxycinnamate | | | |
| MC-DEA: diethanolamine salt of p-methovycinnamatic acid. | | | |
| In vivo SPF: Mean value of determination on 5 volunteers. | | | |
| Expected SPF: Calculated value by adding in vivo SPF-value of 10wt% P3 (4.7) to the in vivo SPF value of 2wt% of the corresponding value for the other filters, divided by 2. | | | |
| Synergy % deviation of in vivo SPF from expected (calculated) SPF. | | | |

The combination of PBSA and the polysiloxane according to the invention shows an unproportional increase of the SPF. Usually, the SPF of filter combinations is very close to the calculated value from the performance of the single filters, therefore, a synergistic effect is shown, in this combination.

## Claims

1. Cosmetic light screening composition containing in an aqueous phase about 0.5 to 10 wt% of 2-phenylbenzimidazol-sulphonic acid or a salt thereof and at least one fatty phase, comprising about 1 to 30 wt% of a linear or cyclic polysiloxane compound of the general formula la or Ib, 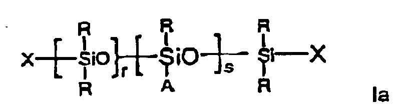 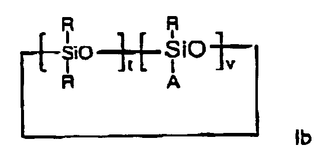 wherein
X signifies R or A;
A signifies a group of the formula IIa, IIb or IIc; 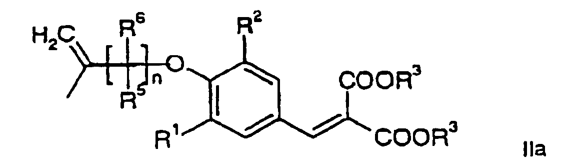 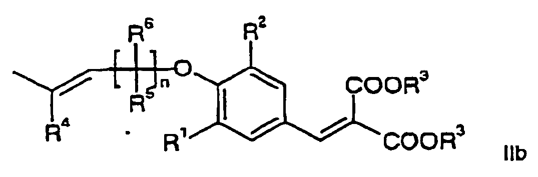 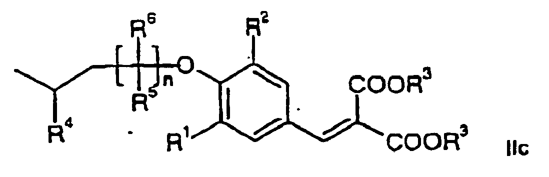
R signifies hydrogen, C₁₋₆-alkyl or phenyl;
R¹ and R² each independently signify hydrogen, hydroxy, C₁₋₆-alkyl or C₁₋₆-alkoxy;
R³ signifies C₁₋₆-alkyl;
R⁴ signifies hydrogen or C₁₋₆-alkyl:
R⁵ and R⁶ each independently signify hydrogen or C₁₋₆-alkyl;
r has a value of from 0 to 250;
s has a value of from 0 to 20;
r+s has a value of at least 3;
t has a value of from 0 to 10;
v has a value of from 0 to 10; and
v+t has a value of at least 3;
n has a value from 1 to 6;
with the proviso that in the case that s is 0 at least one X is A.
and with the further proviso that a sun protecting lotion consisting of the following components
| | |
|---|---|
| Artacel® P135 | 3,00 |
| Magnesium stearate | 0,05 |
| Butylene glycol | 5,00 |
| Elfacos® C26 | 1,00 |
| Magnesium sulfate | 0.50 |
| Isohexadecane | 7,00 |
| Capric/Caprylic Triglyceride | 5,00 |
| Catearylisononanoate | 7,00 |
| Polymer filter X | 7,00 |
| Eusolex® 232 | 3.00 |
| Parsol® 1789 | 2,00 |
| Eusolex® 6300 | 4.00 |
| Uvinul®T150 | 5,00 |
| Preservative | q.s. |
| Perfume | q.s. |
| Water, demin. | ad 100,00 |
is excluded,
wherein polymer filter X is a compound of structure 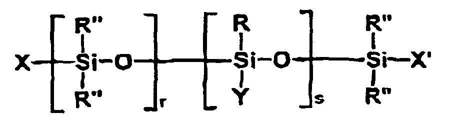 or 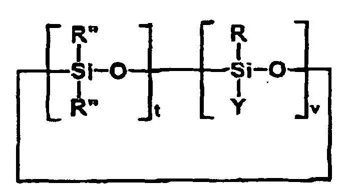 wherein R and R" are methyl, X and X' represent either organic residues such as R and R" or residues Y, r is 0 to 100, s is 0 to 20, t is 0 to 10, v is 1 to 10, wherein v + t is equal or higher than 3 and Y is a residue 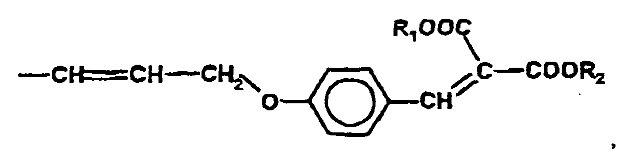 wherein R₁ and R₂ are ethyl and the stoichiometric ratio of Si-O- moieties having chromophore groups Y to Si-O- groups having no chromophore groups Y is about 1:15, and wherein at least one of residues X and X' is a residue Y if s is 0.

2. Cosmetic light screening composition according to claim 1, wherein A is a residue of the formula IIa or IIb.

3. Cosmetic light screening composition according to claim 1 or 2, wherein the ratio of organosiloxane compounds having a chromophore residue of the formula IIa to organosiloxane compounds having a chromophore residue of the formula IIb is about 1:1 to about 19:1, preferably about 2:1 to about 9:1, more preferably about 4:1.

4. Cosmetic light screening composition according to any one of claims 1 to 3, wherein R is methyl.

5. Cosmetic light screening composition according to any one of claims 1 to 4, wherein R¹ and R² are hydrogen, methoxy or ethoxy, or one of R¹ and R² is hydrogen and the other is methyl, methoxy or ethoxy.

6. Cosmetic light screening composition according to any one of claims 1 to 5, wherein R³ is methyl or ethyl.

7. Cosmetic light screening composition according to any one of claims 1 to 6, wherein R⁴ is hydrogen or methyl; R⁵ and R⁶ are hydrogen and n is 1.

8. Cosmetic light screening composition according to any one of claims 1 to 7 containing in an aqueous phase about 0.5 to 5 wt% of 2-phenylbenzimidazol-sulphonic acid or a salt thereof, and at least one fatty phase, comprising about 2 to 20 wt% of a linear polysiloxane compound of the general formula Ia, wherein
X signifies methyl;
A signifies a group of the formula IIa or IIb;
R signifies methyl;
R¹ and R² signify hydrogen, methoxy or ethoxy, or one of R¹ and R² is hydrogen and the other is methyl, methoxy or ethoxy.
R³ signifies methyl or ethyl;
R⁴ signifies hydrogen or methyl;
R⁵ and R⁶ signify hydrogen;
r is about 5 to about 150;
s is about 2 to about 10;
n has a value of 1.

9. Cosmetic light screening composition according to claim 8, containing in an aqueous phase about 1 to 2 wt% of 2-phenylbenzimidazol-sulphonic acid or a salt thereof and at least one fatty phase, comprising about 5 wt% of a linear polysiloxane compound of the general formula la, wherein
R¹ and R² signify hydrogen;
R³ signifies ethyl;
R⁴ signifies hydrogen;
r is a statistical mean of about 4;
s is a statistical mean of about 60.

10. Cosmetic light screening composition according to any one of claims 1 to 9, wherein said composition contains in addition common UV-A filter and /or UV-B filters.

11. The use of a cosmetic light screening composition according to any one of claims 1 to 10 as absorber for the ultraviolet light.

## Patentansprüche

1. Kosmetische Lichtschutzzusammensetzung, enthaltend in einer wässerigen Phase etwa 0,5 bis 10 Gew.-% 2-Phenylbenzimidazol-sulfonsäure oder ein Salz davon und mindestens eine Fettphase, umfassend etwa 1 bis 30 Gew.-% einer linearen oder cyclischen Polysiloxanverbindung der allgemeinen Formel Ia oder Ib 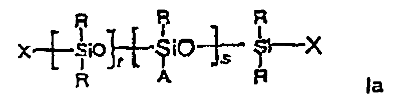 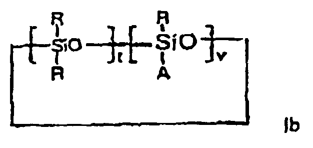 worin
X R oder A darstellt;
A eine Gruppe der Formel IIa, IIb oder IIc darstellt 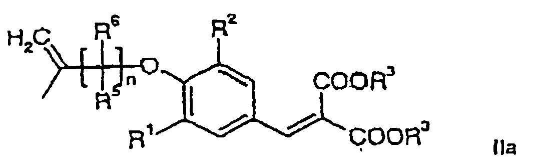 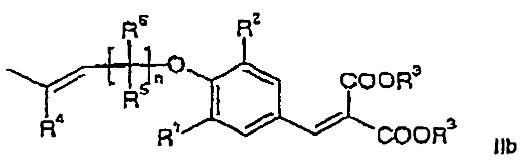 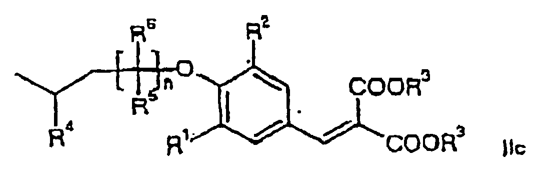 R Wasserstoff, C₁₋₆-Alkyl oder Phenyl darstellt;
R¹ und R² jeweils unabhängig Wasserstoff, Hydroxy, C₁₋₆-Alkyl oder C₁₋₆-Alkoxy darstellen;
R³ C₁₋₆-Alkyl darstellt;
R⁴ Wasserstoff oder C₁₋₆-Alkyl darstellt;
R⁵ und R⁶ jeweils unabhängig Wasserstoff oder C₁₋₆-Alkyl darstellen;
r einen Wert von 0 bis 250 aufweist;
s einen Wert von 0 bis 20 aufweist;
r + s einen Wert von mindestens 3 aufweist;
t einen Wert von 0 bis 10 aufweist;
v einen Wert von 0 bis 10 aufweist; und
v + t einen Wert von mindestens 3 aufweist;
n einen Wert von 1 bis 6 aufweist;
mit der Maßgabe, daß in dem Fall, wo s 0 ist, mindestens ein X A ist,
und mit der weiteren Maßgabe, daß eine Sonnenschutzlotion, die aus den folgenden Komponenten besteht,
| | |
|---|---|
| Artacel® P135 | 3,00 |
| Magnesiumstearat | 0,05 |
| Butylenglykol | 5,00 |
| Elfaco® C26 | 1,00 |
| Magnesiumsulfat | 0,50 |
| Istihexadecan | 7,00 |
| Caprin-/Capryl-Triglycerid | 5,00 |
| Cetearylisononanoat | 7,00 |
| Polymerfilter X | 7,00 |
| Eusolex®232 | 3,00 |
| Parsol®1789 | 2,00 |
| Eusolex®6300 | 4,00 |
| Uvinul®T150 | 5,00 |
| Konservierungsmittel | q.s. |
| Duftstoff | q.s. |
| vollentsalztes Wasser | auf 100,00 |
ausgeschlossen ist,
worin der Polymerfilter X eine Verbindung mit der Struktur 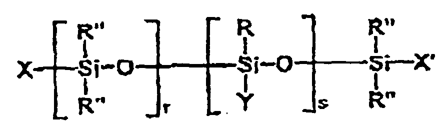 oder 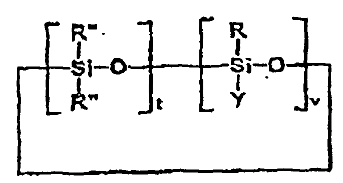 ist,
worin R und R" Methyl sind, X und X' entweder organische Reste, wie R und R", oder Reste Y darstellen, r 0 bis 100 ist, s 0 bis 20 ist, t 0 bis 10 ist, v 1 bis 10 ist, worin v + t gleich oder höher als 3 ist und Y ein Rest 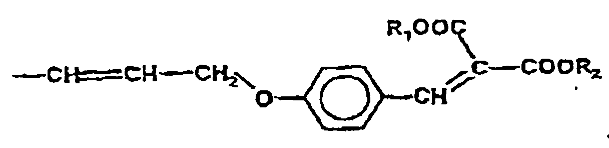 ist,
worin
R₁ und R₂ Ethyl sind und das stöchiometrische Verhältnis von Si-O-Komponenten mit Chromophorgruppen Y zu Si-O-Gruppen ohne Chromophorgruppen Y etwa 1 : 15 beträgt, und worin mindestens einer der Reste X und X' ein Rest Y ist, wenn s 0 ist.

2. Kosmetische Lichtschutzzusammensetzung nach Anspruch 1, wobei A ein Rest der Formel IIa oder IIb ist.

3. Kosmetische Lichtschutzzusammensetzung nach Anspruch 1 oder 2, wobei das Verhältnis der Organosiloxanverbindungen mit einem Chromophorrest der Formel IIa zu den Organosiloxanverbindungen mit einem Chromophorrest der Formel IIb etwa 1 : 1 bis etwa 19 : 1, vorzugsweise etwa 2 : 1 bis etwa 9 : 1, stärker bevorzugt etwa 4 : 1 beträgt.

4. Kosmetische Lichtschutzzusammensetzung nach einem der Ansprüche 1 bis 3, wobei R Methyl ist.

5. Kosmetische Lichtschutzzusammensetzung nach einem der Ansprüche 1 bis 4, wobei R¹ und R² Wasserstoff, Methoxy oder Ethoxy sind, oder einer von R¹ und R² Wasserstoff ist und der andere Methyl, Methoxy oder Ethoxy ist.

6. Kosmetische Lichtschutzzusammensetzung nach einem der Ansprüche 1 bis 5, wobei R³ Methyl oder Ethyl ist.

7. Kosmetische Lichtschutzzusammensetzung nach einem der Ansprüche 1 bis 6, wobei R⁴ Wasserstoff oder Methyl ist; R⁵ und R⁶ Wasserstoff sind und n 1 ist.

8. Kosmetische Lichtschutzzusammensetzung nach einem der Ansprüche 1 bis 7, enthaltend in einer wässerigen Phase etwa 0,5 bis 5 Gew.-% 2-Phenylbenzimidazol-sulfonsäure oder ein Salz davon und mindestens eine Fettphase, umfassend etwa 2 bis 20 Gew.-% einer linearen Polysiloxanverbindung der allgemeinen Formel Ia, wobei
X Methyl darstellt;
A eine Gruppe der Formel IIa oder IIb darstellt;
R Methyl darstellt;
R¹ und R² Wasserstoff, Methoxy oder Ethoxy darstellen, oder einer von R¹ und R² Wasserstoff ist und der andere Methyl, Methoxy oder Ethoxy ist;
R³ Methyl oder Ethyl darstellt;
R⁴ Wasserstoff oder Methyl darstellt;
R⁵ und R⁶ Wasserstoff darstellen;
r etwa 5 bis etwa 150 beträgt;
s etwa 2 bis etwa 10 beträgt;
n einen Wert von 1 aufweist.

9. Kosmetische Lichtschutzzusammensetzung nach Anspruch 8, enthaltend in einer wässerigen Phase etwa 1 bis 2 Gew.-% 2-Phenylbenzimidazol-sulfonsäure oder ein Salz davon und mindestens eine Fettphase, umfassend etwa 5 Gew.-% einer linearen Polysiloxanverbindung der allgemeinen Formel Ia, wobei
R¹ und R² Wasserstoff darstellen;
R³ Ethyl darstellt;
R⁴ Wasserstoff darstellt;
r ein statistisches Mittel von etwa 4 ist;
s ein statistisches Mittel von etwa 60 ist.

10. Kosmetische Lichtschutzzusammensetzung nach einem der Ansprüche 1 bis 9, wobei die Zusammensetzung außerdem bekannte UV-A-Filter und/oder UV-B-Filter enthält.

11. Verwendung einer kosmetischen Lichtschutzzusammensetzung nach einem der Ansprüche 1 bis 10 als Absorber für UV-Licht.

## Revendications

1. Composition cosmétique d'écran solaire contenant, dans une phase aqueuse, environ 0,5% à 10% en poids d'acide 2-phénylbenzimidazolsulfonique ou d'un sel de celui-ci et au moins une phase grasse comprenant environ 1% à 30% en poids d'un composé polysiloxane linéaire ou cyclique de formule générale Ia ou Ib 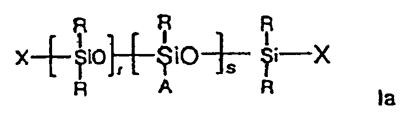 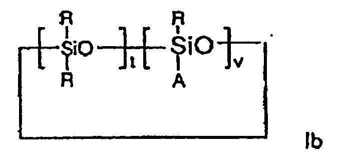 dans lesquelles
X représente R ou A ;
A représente un groupe de formule IIa, IIb ou IIc 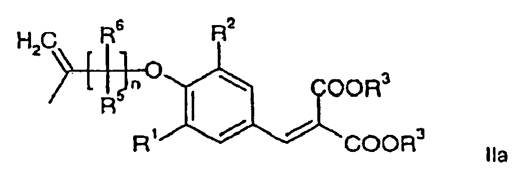 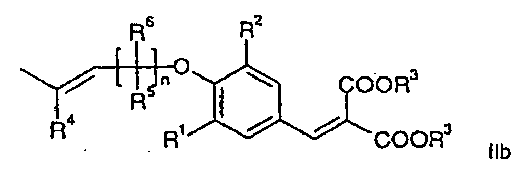 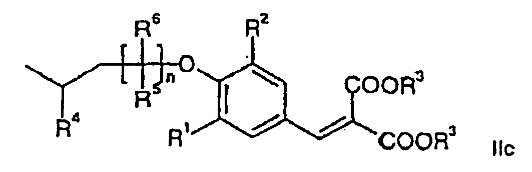
R représente un atome d'hydrogène, un groupe alkyle en C₁ à C₆ ou un groupe phényle ;
R¹ et R² représentent chacun, indépendamment l'un de l'autre, un atome d'hydrogène, un groupe hydroxy, un groupe alkyle en C₁ à C₆ ou un groupe alcoxy en C₁ à C₆ ;
R³ représente un groupe alkyle en C₁ à C₆ ;
R⁴ représente un atome d'hydrogène ou un groupe alkyle en C₁ à C₆ ;
R⁵ et R⁶ représentent chacun, indépendamment l'un de l'autre, un atome d'hydrogène ou un groupe alkyle en C₁ à C₆ ;
r vaut 0 à 250 ;
s vaut 0 à 20 ;
r + s vaut au moins 3 ;
t vaut 0 à 10 ;
v vaut 0 à 10 ; et
v + t vaut au moins 3 ;
n vaut 1 à 6 ;
à condition que, lorsque s vaut 0, au moins un X représente A
et également à condition qu'une lotion de protection solaire constitué des composants suivants
| | |
|---|---|
| Arlacel® P135 | 3,00 |
| Stéarate de magnésium | 0,05 |
| Butylèneglycol | 5,00 |
| Elfacos® C26 | 1,00 |
| Sulfate de magnésium | 0,50 |
| Isohexadécane | 7,00 |
| Trigylcéride caprique/caprylique | 5,00 |
| Isononanoate de cétéaryl | 7,00 |
| Filtre X polymère | 7,00 |
| Eusolex® 232 | 3,00 |
| Parsol® 1789 | 2,00 |
| Eusolex® 6300 | 4,00 |
| Uvinul® T150 | 5,00 |
| Conservateur | q.s. |
| Parfum | q.s. |
| Eau déminéralisée | jusqu'à 100,00 |
soit exclu,
dans lequel le filtre X polymère est un composé de structure 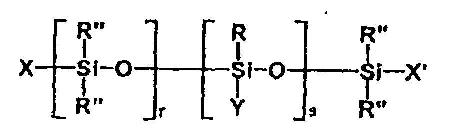 ou 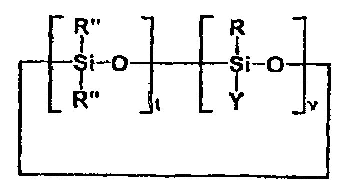 dans lesquelles R et R" représentent des groupes méthyle, X et X' représentent soit des groupes organiques tels que R et R", soit des groupes Y, r vaut 0 à 100, s vaut o à 20, t vaut 0 à 10, v vaut 1 à 10, v + t étant supérieur ou égal à 3 et Y représente un groupe 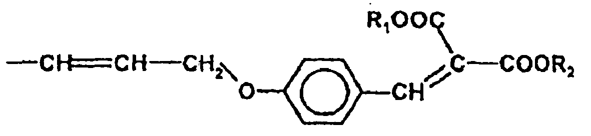 dans lequel R₁ et R₂ représentent des groupes éthyle et le rapport stoechiométrique des fractions Si-O- comprenant des groupes chromophores Y aux fractions Si-O- ne comprenant pas de groupes chromophores Y est d'environ 1:15, et dans lesquelles au moins l'un des groupes X et X' représente un groupe Y lorsque s vaut 0.

2. Composition cosmétique d'écran solaire selon la revendication 1, dans laquelle A représente un groupe de formule IIa ou IIb.

3. Composition cosmétique d'écran solaire selon la revendication 1 ou 2, dans laquelle le rapport des composés organosiloxane comprenant un groupe chromophore de formule IIa aux composés organosiloxane comprenant un groupe chromophore de formule IIb est d'environ 1:1 à environ 19:1, de préférence d'environ 2:1 à environ 9:1, de manière plus particulièrement préférée d'environ 4:1.

4. Composition cosmétique d'écran solaire selon l'une quelconque des revendications 1 à 3, dans laquelle R représente un groupe méthyle.

5. Composition cosmétique d'écran solaire selon l'une quelconque des revendications 1 à 4, dans laquelle R¹ et R² représentent des atomes d'hydrogène, des groupes méthoxy ou éthoxy, ou l'un de R¹ et de R² représente un atome d'hydrogène et l'autre représente un groupe méthyle, méthoxy ou éthoxy.

6. Composition cosmétique d'écran solaire selon l'une quelconque des revendications 1 à 5, dans laquelle R³ représente un groupe méthyle ou éthyle.

7. Composition cosmétique d'écran solaire selon l'une quelconque des revendications 1 à 6, dans laquelle R⁴ représente un atome d'hydrogène ou un groupe méthyle ; R⁵ et R⁶ représentent des atomes d'hydrogène et n vaut 1.

8. Composition cosmétique d'écran solaire selon l'une quelconque des revendications 1 à 7 contenant, dans une phase aqueuse, environ 0,5% à 5% en poids d'acide 2-phénylbenzimidazolsulfonique ou d'un sel de celui-ci et au moins une phase grasse comprenant environ 2% à 20% en poids d'un composé polysiloxane linéaire de formule générale Ia, dans laquelle
x représente un groupe méthyle ;
A représente un groupe de formule IIa ou IIb ;
R représente un groupe méthyle ;
R¹ et R² représentent des atomes d'hydrogène, des groupes méthoxy ou éthoxy, ou l'un de R¹ et de R² représente un atome d'hydrogène et l'autre représente un groupe méthyle, méthoxy ou éthoxy ;
R³ représente un groupe méthyle ou éthyle ;
R⁴ représente un atome d'hydrogène ou un groupe méthyle ;
R⁵ et R⁶ représentent des atomes d'hydrogène ;
r vaut environ 5 à environ 150 ;
s vaut environ 2 à environ 10 ;
n vaut 1.

9. Composition cosmétique d'écran solaire selon la revendication 8 contenant, dans une phase aqueuse, environ 1% à 2% en poids d'acide 2-phénylbenzimidazolsulfonique ou d'un sel de celui-ci et au moins une phase grasse comprenant environ 5% en poids d'un composé polysiloxane linéaire de formule générale Ia, dans laquelle
R¹ et R² représentent des atomes d'hydrogène ;
R³ représente un groupe éthyle ;
R⁴ représente un atome d'hydrogène ;
r est une moyenne statistique d'environ 4 ;
s est une moyenne statistique d'environ 60.

10. Composition cosmétique d'écran solaire selon l'une quelconque des revendications 1 à 9, dans laquelle ladite composition contient en outre des filtres UV-A et/ou UV-B classiques.

11. Utilisation d'une composition cosmétique d'écran solaire selon l'une quelconque des revendications 1 à 10 en tant qu'absorbeur de la lumière ultraviolette.
